(19) **Europäisches Patentamt**

**European Patent Office**

**Office européen des brevets**

(11) **EP 0 608 987 B1**

(12) **EUROPEAN PATENT SPECIFICATION**

(45) Date of publication and mention
of the grant of the patent:
**10.10.2001 Bulletin 2001/41**

(51) Int Cl.[7]: **G01N 33/574**, G01N 33/50

(21) Application number: **94300269.1**

(22) Date of filing: **14.01.1994**

(54) **Method for detecting rare events**

Verfahren zum Nachweis von seltenen Ereignissen

Méthode pour la détection d'événements rares

(84) Designated Contracting States:
**BE DE ES FR GB IT**

(30) Priority: **26.01.1993 US 9185**

(43) Date of publication of application:
**03.08.1994 Bulletin 1994/31**

(73) Proprietor: **Becton, Dickinson and Company**
**Franklin Lakes, New Jersey 07417-1880 (US)**

(72) Inventors:
• **Gross, Hans-Joachim**
**Frankfurt (DE)**

• **Verwer, Ben J.H.**
**Santa Clara, Ca 95729 (US)**

(74) Representative: **Ruffles, Graham Keith**
**MARKS & CLERK,**
**57-60 Lincoln's Inn Fields**
**London WC2A 3LS (GB)**

(56) References cited:
**EP-A- 0 132 064**      **EP-A- 0 347 039**
**EP-A- 0 347 210**      **EP-A- 0 470 810**
**EP-A- 0 509 718**      **EP-A- 0 552 707**
**WO-A-93/18068**

## Description

Field of the Invention

This invention relates to a method for the detection of rare events in a sample of particles, and more particularly relates to a method for the detection of rare cells in a sample of cells at frequencies of less than one in $10^6$ cells.

Background of the Invention

Particle analysis generally comprises the analysis of cells, nuclei, chromosomes and other particles for the purpose of identifying the particles as members of different populations and/or sorting the particles into different populations. This type of analysis includes automated analysis by means such as flow cytometry. In flow cytometry, the particle, such as a cell, may be labeled with one or more markers and then examined for the presence or absence of such markers. In the case of a cell, such as a leukocyte, tumor cell or microorganism, the marker can be directed to molecules on the cell surface, such as receptor proteins, or to molecules in the cytoplasm or nucleus such as proteins, enzymes or nucleic acids. Examination of a particle's physical characteristics (e.g., size), as well as for the presence or absence of marker(s) can provide additional information which can be useful in identifying the population to which a particle belongs.

Flow cytometry comprises a well known methodology using multi-parameter data for identifying and distinguishing between different particle types in a sample. For example, a sample of cellular particles may be drawn from a variety of biological fluids such as blood, lymph, bone marrow, ascites, pericardial fluid, pleural fluid, cerebrospinal fluid or urine, or may be derived from suspensions or aspirates of cells from organs and solid tissues such as brain, GI tract, prostate, lung, breast, kidney, lymph node or liver. In a flow cytometer, cells are passed substantially one at a time through one or more sensing regions where in each region each cell is illuminated by an energy source. The energy source generally comprises an illumination means that emits light of a single wavelength such as that provided by a laser (e.g., He/Ne or argon ion) or a mercury arc lamp with appropriate bandpass filters. Light at 488nm is a presently preferred wavelength of excitation when a single light source is used and 632nm is preferred as a second light source when two light sources are used.

In series with one or more sensing regions, multiple light collection means, such as photomultiplier tubes, are used to record light that passes through each cell (generally referred to as forward light scatter), light that is reflected orthogonal to the direction of flow of the cells through the sensing region (generally referred to as orthogonal or side light scatter) and fluorescent light that may be emitted from the cell, if it is labeled with fluorescent marker(s), as it passes through the sensing region and is illuminated by the energy source. Each of forward light scatter (or FSC), orthogonal light scatter (SSC), and fluorescence emissions (FL1, FL2, etc.) comprise a separate parameter for each cell (or each "event"). Thus, for example, up to four parameters can be collected (and recorded) from a cell labeled with two different fluorescence markers (i.e., two scatter parameters and two fluorescent parameters per cell).

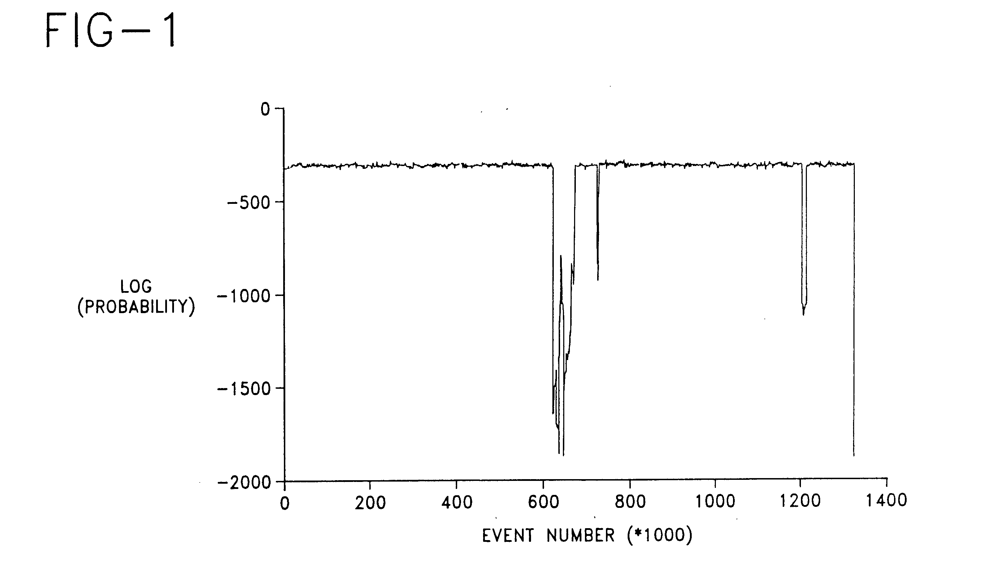

Flow cytometers further comprise data acquisition, analysis and storage means such as a computer equipped with appropriate software. Signals from each PMT are converted into multiple data channels and stored by the computer. The purpose of the analysis system is to classify and count cells wherein each cell presents itself as a set of digitized parameter values. Typically, by current analysis methods, data collected in real time (or recorded for later analysis) is plotted in 2-D space for ease of visualization. Such plots are referred to as dot plots and a typical example of a dot plot drawn from light scatter data recorded for leukocytes is shown in FIG. 1 of U.S. Pat. No. 4,987,086. By plotting orthogonal light scatter versus forward light scatter, one can distinguish between granulocytes, monocytes and lymphocytes in a population of leukocytes isolated from whole blood. By electronically (or manually) gating on only lymphocytes using light scatter, for example, and by the use of the appropriate monoclonal antibodies labeled with fluorochromes of different emission wavelength, one can further distinguish between cell types within the lymphocyte population (e.g., between T helper cells and T cytotoxic cells). U.S. Pat. Nos. 4,727,020, 4,704,891, 4,599,307 and 4,987,086 describe the arrangement of the various components that comprise a flow cytometer, the general principles of use and one approach to gating on cells in order to discriminate between populations. EP 347,210 describes a method for multi-parameter analysis of cells using at least two nucleic acid dyes and at least one fluorescently labelled cell surface maker.

While the above-described methods are useful and accurate for the analysis of particles that occur in a sample at a frequency of greater than 1%, accuracy is much less reliable in the analysis of particles that occur with lesser frequency. These events are termed "rare events."

Accuracy in the counting and analysis of rare events is important. For example, in many cancers elimination of "cancer cells" is the desired goal of a therapy. It is important to know if all of the cancer cells have been eliminated. If a number of cancer cells remain but are undetected because existing instrumentation cannot reliably detect the cells that remain because of their low frequency, a patient could be given an erroneous "clean bill of health." Similarly, if these minimum residual cells have not been eliminated, it is important for monitoring purposes to detect increases in the frequency of such cells at the earliest possible time in order to determine whether changes in treatment are required. Thus, accurately quantitating the number of cancer cells that remain after treatment is important in the future treatment and monitoring of cancer.

A concern in the accurate counting of cells by flow cytometry is assuring that an event which the instrument senses as being positive for a "fluorescent" marker actually is so as a result of being labelled with a fluorescent marker. It has been reported that freshly isolated unlabelled mouse bone marrow contains autofluorescent particles that a cytometer senses as being "fluorescent." The incidence of these events was reported as one per $10^4$ cells. As a result, it has been considered that the lowest achievable level of detection using commercially available cytometers is about one cell in $10^5$.

In addition to unlabelled cells being recorded at some frequency as "fluorescent" events, the use of immunofluorescence markers can cause additional errors in data analysis. Monoclonal antibodies labelled with fluorescent dyes have a high affinity for their antigen. Unfortunately, because of the nature and structure of the cells on which such antigens appear, immunofluorescence markers will bind to structures other than the intended antigen. Thus, irrelevant cells can be recorded as positive fluorescent events due to non-specific labelling. For cells whose frequency is below 1% of the cells in a sample, this is an additional problem to be addressed.

A third problem with conventional cytometry is the speed at which cells are analyzed. Current instrumentation operates at an acquisition rate of 5,000 to 10,000 events per second. Current instrumentation also has a limited random access memory associated with the data analysis hardware to acquire events for real time analysis. Thus, to acquire about $10^8$ events, it takes about 3 to 6 hours at a minimum. To maintain cells over such a long period of time with the same fluorescent characteristics, it is necessary to fix the cells. Fixation will have some effect on the physical characteristics of the cell as opposed to fresh living cells, and thus, presents a further opportunity for the introduction of error into the system.

Accordingly, an improved method for the analysis of cells is required if rare event analysis is to be a more useful and accurate tool in cytometry.

Summary of the Invention

This invention comprises a high-speed method for the detection of particles in a sample at frequencies of less than one in $10^4$.

The particles can be cells in biological fluids, including red and white blood cells, dis-aggregated cells from organs and solid tissues and microorganisms. Samples of such cells can be taken from blood, urine, bone marrow, ascites, pericardial fluid, pleural fluid, cerebrospinal fluid, lymph and other biological fluids, as well as from organs and solid tissues such as brain, kidney, prostate, liver, spleen, lung, breast, lymph node and GI tract.

The particles to be analyzed are combined with at least one first marker that is specific for (i.e., has a high binding affinity for) the particles to be detected and at least one second marker that is specific for the majority of the remainder of the particles in the sample but is negative for (i.e., has low or no binding affinity for) the particles to be detected. It should be appreciated that in few circumstances will there be a single marker which will be specific for the majority of remaining particles in a sample. Therefore, most uses will require more than one second marker. This second marker also can be referred to as the exclusion marker.

Where the particles comprise cells, it is preferred that the markers be immunofluorescence markers. Where more than one immunofluorescence marker is used, it is preferred that each of the first markers be labelled-with a different fluorescent dye. Each such dye should have an emission wavelength that is distinguishable from the other(s). It further is preferred that the second marker be labelled with a fluorescent dye that has an emission wave length distinguishable from that of the dyes used in connection with the first marker(s). It should be noted, however, that while each of the first markers generally is labelled with a different dye (when multiple first markers are used), when more than one second marker is used they all preferably are labelled with the same dye. Finally, it is to be appreciated that where multiple markers are used they can comprise combinations of immunofluorescence markers and other cytoplasmic or nuclear markers (e.g., nucleic acid dyes).

In the preferred embodiment, the number of first markers is two, and the number of second markers is greater than one.

This invention has particularly utility in the detection of cancer cells in bone marrow or peripheral blood preparations of stem cells. Stem cells have been described as being $CD34^+$. Subsets of such cells include cells that are $CD34^+/CD38^-$, cells that are $CD34^+/CD38^-/HLA-DR^+$ and cells that are $CD34^+/CD38^-/HLA-DR^-$. In the treatment of patients requiring infusion of stem cells, it is important to eliminate any remaining cancer cells from the preparation. For those cancers that are $CD34^-$, antibodies against CD34 would comprise the exclusion color while antibodies specific for the cancer cells would comprise the first marker(s).

Brief Description of the Drawings

FIG. 1 is a plot of natural log probability of each sequence of 1000 events for a sample of 140 x $10^6$ cells containing rare events at a frequency of about $10^{-6}$. Each of the probabilities is calculated using the relative frequencies of parameter values in accordance with the algorithm set forth below.

FIG. 2 is a schematic diagram of the gates applied to n-dimensional data (and shown in 2-D form) in order to isolate rare events.

FIG. 3 comprises a series of dot plots of (A) - (G) SSC versus FSC, (H) FL2 versus FL1 and (I) FL2 versus FL3 for various amounts of REH cells seeded into $10^8$ PBMC.

Detailed Description of the Invention

Peripheral blood mononuclear cells ("PBMC") were isolated from buffy coats by density gradient separation. After two washes with 0.45μ filtered $Ca^{2+}$ and $Mg^{2+}$ free phosphate buffered saline ("PBS"), the cells were resuspended in 0.45μ filtered PBS in 20% fetal calf serum ("FCS"). Cell suspensions of more than $1.2 \times 10^9$ cells were rotated for one hour. Viability was determined by the propidium-iodide exclusion test on a FACScan brand flow cytometer (Becton Dickinson Immunocytometry Systems, "BDIS"). Viability was always greater than 98%.

Cells not stained were counted on a hemocytometer. $2.5 \times 10^8$ PBMC were aliquoted in up to 4 separate 15ml tubes. Each tube was supplemented 0.45μ filtered FCS to a total volume of 14ml.

The pre-B cell line REH was cultured in RPMI in 10% FCS in an atmosphere of 7% $CO_2$ at 37°C. Twelve hours prior to use, the cultures were split and fresh medium was added. The cells were harvested and washed with 0.45μ filtered PBS prior to sort. Viability was determined as above. Viability was always greater than 94%.

Where required, pre-B cells were labelled with immunofluorescence markers as follows. Pre-B cells were sorted as described below into $2.5 \times 10^8$ PBMC. The mixture then was rotated for one hour to ensure dispersion. The cells then were spun down and supernatant removed to leave about 50μl of solution. 50μl of normal mouse serum (Dako) was added. The cells were resuspended and rotated for one hour.

190μl of labelling cocktail was added. The cocktail contained 25% normal mouse serum and the following antibodies at the following final sample concentrations. To separate pre-B cells from mature B cells, anti-CD19 fluorescein isothiocyanate (Leu 12 FITC, BDIS) 4.6μg/ml and anti-CD20 FITC (Leu 16 FITC, BDIS) 2.4μg/ml were added as a first marker. To exclude T cells, granulocytes, monocytes and platelets, anti-CD7 peridinin chlorophyll protein (Leu 9 PerCp, BDIS) 22.9μg/ml, anti-CD3 PerCp (Leu 4 PerCp, BDIS) 5.4μg/ml, anti-CD4 PerCp (Leu 3a PerCp, BDIS) 1.6μg/ml, anti-CD8 PerCp (Leu 2a PerCp, BDIS) 2.0μg/ml, anti-CD15 PerCp (Leu M1 PerCp, BDIS) 4.7μg/ml, anti-CD15 PerCp (L-16 PerCp) 6.4 μg/ml, anti-CD14 PerCp (Leu M3 PerCp, BDIS) 1.3μ g/ml, anti-CD41a PerCp 12.3μg/ml and anti-CD42b PerCp 29.5μ g/ml were added as second markers. For additional identification of pre-B cells, anti-CD10 phycoerythrin (anti-CALLA PE, BDIS) 6.2 μg/ml was added as an additional first marker.

As an isotype control, anti-KLH PE 6.2μg/ml was added.

The cells then were resuspended and rotated for one hour to ensure complete labelling. The cells were washed twice in PBS and fixed in a total volume of 200ml in 0.5% paraformaldehyde for 12 hours. After harvesting, the cells were washed once in PBS and finally resuspended in a total volume of 45ml PBS/0.25% FCS. The yield of recovered PBMC was $58.2 \pm 12.5\%$.

All cells were analyzed on either a FACScan brand flow cytometer or a FACStar$^{Plus}$ brand cell sorter (BDIS). The instruments were equipped with 488nm argon lasers. The cell sorter was further equipped with a 70μ nozzle with a drop drive frequency of 27 kHz with 3 drop envelopes.

Data acquisition and analysis was performed on a Hewlett Packard 340 computer with 16Mb of RAM using Lysis software (BDIS). Pulse width measurements were used to discriminate single cells from cell aggregates.

Prior to any experiment being run, each instrument was cleaned to assure removal of particles and debris from prior experiments. This process is described in U.S. Patent No. 5,087,295. Briefly, a mixture of 0.07M NaOCl and 0.05M NaOH in particle free deionized water were added to the sheath tank. The instrument then was run for 20 minutes by-passing the built-in 0.2μ sheath fluid filter. This tank then was emptied and particle free deionized water was added and the instrument then run for 30 minutes. The tank was again emptied and sheath fluid then was added to the tank. The fluidics system was allowed to equilibrate for 15 minutes thereafter.

Electronic stability of the FACScan instrument was checked by a continuous run for 102 hours. The instrument has a test mode which sends sequenced light pulses (approximately 2000/second) to all PMTs. A live gate was set in FL1 and FL2 in accordance with manufacturer's instructions. This gate excluded all events at or below the light pulse intensities and indicates spurious events generated by the electronics of the instrument. No spurious events were generated in the test run.

For rare event acquisition, approximately $10^8$ PBMC were run on an instrument at an acquisition rate of 5,000 to 6,000 events per second. Event acquisition was triggered on a threshold set in FL2. The threshold was set just above the upper limit of autofluorescence of the bulk of the negative cells. A strong FL2 signal on possible rare events was preferred to increase the probability of detection of rare events. It should be appreciated that fluorescence thresholds may be set for other fluorescence parameters as well in order to further refine the selection method. In addition, a live gate was set in forward and side scatter which rejects most of the very small particles such as platelets. These procedures reduce the number of events taken to RAM and to the system's hard disc to about 1%. On average, $10^6$ events were recorded for about $10^8$ cells analyzed.

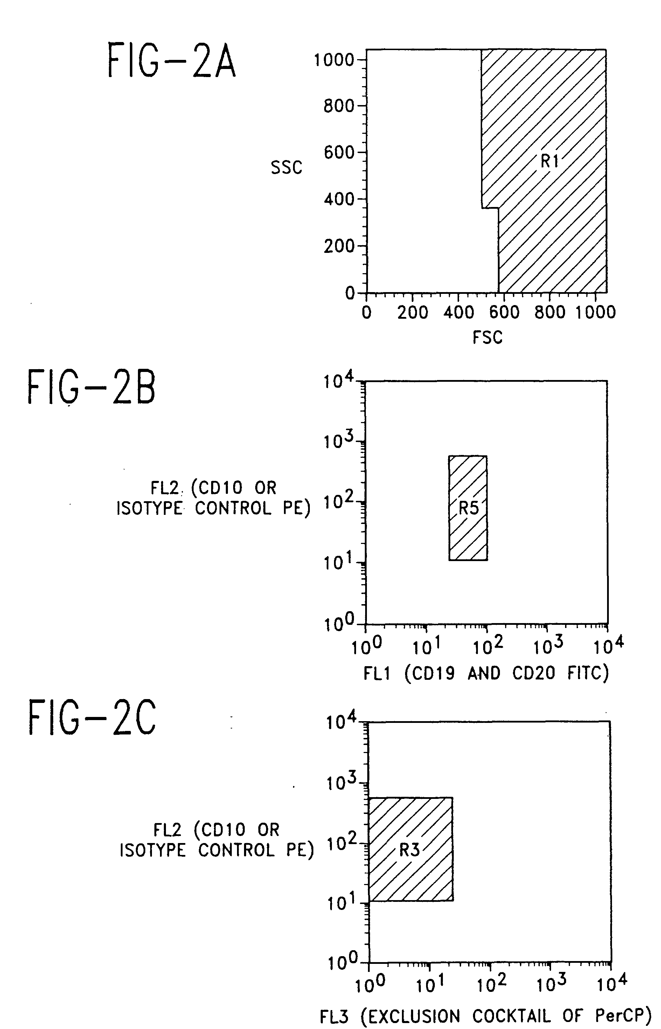

Prior to the analysis of the acquired data file, an algorithm was applied to remove bursts of events. FIG. 1 displays event number versus the natural log of the probability of each sequence of 1000 events for a sample of 140 x 10⁶ cells containing rare cells at a frequency of 10⁻⁶. The black heavy lines at the top of the figure represent rare events acquired as defined by the gates described in FIG. 2.

Three major bursts are apparent. The bursts of events were not of a Poisson distribution, and thus, represented clumps of particles or debris the inclusion of which would lead to spurious results. In two of the bursts, "rare" events were detected. The algorithm described below removes all events inside of a burst, and thus, excludes those "rare" events from being taken into account. The conjecture is that these events are not real rare events, or might not be, and therefore should be disregarded to assure integrity of the system. It should be readily apparent that this algorithm has special utility for rare event detection but will have general utility to the acquisition and analysis of any data set where fidelity is desired.

The algorithm was applied as follows. Assume a data set with **L** events consisting of **N** parameters digitized in **B** bits. Then each event $E_i$ (**i**=1 .. **L**) will receive a probability $P_i$ based on a number of **W** events in a window around $E_i$. Let $H_{ijk}$ be the number of events in the window around $E_i$ in which the jth components have a value **k** (**i**=1...**L**, **j**=1...**N**, **k**=1...$2^B$). Then $H_{ijk}$ is Poisson distributed with $\lambda_{jk}$=**W**·$P_{jk}$, where $P_{jk}$ is the *a priori* probability that parameter **j** has value **k.** This applies only if $\lambda_{jk}$ is moderate. $P_{jk}$ can be known or estimated. In the later case, it can be estimated during a test run or from the data itself as a ratio of the total number of events with the **k**th component equal to **j** and the total number of events **L**.

Any realization of $H_{ijk}$ has a certain probability associated with it, namely:

$$P_{ijk} = e^{(-\lambda_{jk})} \frac{\lambda_{jk}^{H_{ijk}}}{H_{ijk}!}$$

Multiplying the $P_{ijk}$ over **j** and **k** gives the desired probability $P_i$, if all $P_{ijk}$ were independent (which they are not because the number of events in a window is limited). However, if a fraction **f** of $P_{ijk}$ with **f** << W is taken, then all of the probabilities can be taken as independent when the **f** is spread equally over all possible **j**.

Once all $P_i$ have been found, a minimum filter of size **W** is applied. The minimum filter assigns to each event the minimum value of all the $P_i$ in the window around it. This has the effect of eliminating certain events that occur just before or after a burst.

Finally, a quantile **Q** of all $P_i$ is found. (Ten percent provides an appropriate measure for **Q**.) All events with a $P_i$ less than **Q** are removed from the data set resulting in a data set of length (1-**Q**)*L. It should be noted that the algorithm will still remove **Q** events; however, because the probabilities for these events vary only due to statistical noise, there will be no consequence for data analysis.

Modifications of the method to calculate the probabilities (e.g., considering each event in n-dimensional space) or a direct "is in agreement with" or "is not in agreement with" the distribution as determined from the whole data file. Furthermore, one could use a different method to find the threshold below which events are removed (e.g., visual inspection of the calculated probabilities).

Referring to FIG. 2, as general outline of how first and second markers are used to identify rare events, such as white blood cells, is shown. First, a fluorescence threshold is applied above the autofluorescence background of the cells to exclude all events that do not have an immunofluorescence marker. Second, a region, $R_1$, is found in FSC and SSC that will exclude small particles, such as platelets. See FIG. 2A. It should be appreciated that if the exclusion of small particles is not necessary or may accomplished through the use of the second markers, this step can be omitted. Finally, fluorescence regions, $R_3$ and $R_5$, are found for at least two combinations of the fluorescence data where rare events should occur. In this example, for pre-B cells, the plot is of the two first markers (i.e., FL2 versus FL1) which will contain only labelled B cells (see FIG. 2B), and there is a plot of one first marker versus the exclusion marker(s) (i.e., FL2 versus FL3) which will contain only labelled B cells (see FIG. 2C). A rare event, thus, is defined as a cell exceeding the fluorescence threshold and falling within each of the 3 gates set.

It should be apparent to one of ordinary skill that these displays are not necessary for data analysis, but are provided as a means to visualize the steps included in the method of the invention. These steps generally are run without the need for the user to see them. It also should be apparent that as the number of first markers increases, so too will the number of gates set for the various combinations of first markers.

The following examples described in more detail the nature of this invention.

Example I

250, 2,500 and 25,000 pre-B cells were sorted into 2.5 x 10⁸ PBMC. This represents a concentration of pre-B cells of 10⁻⁶, 10⁻⁵ and 10⁻⁴ respectively. Sorting was done using a FACStar^Plus cell sorter using the instrument's "count mode" and "pulse processor." Count mode delivers a pre-set number of cells with pre-set characteristics. The pulse processor measures pulse width which is used to discriminates single cells from aggregates.

Prior to sorting, sort accuracy was checked as follows. An aliquot of the pre-B cells was labelled with nuclear UV excitable dye Hoechst 33 342 (10µg/ml) for 10 minutes at 37° C for examination of cells under a fluorescence microscope. After washing and suspending the pre-B cells in PBS, a defined number of cells were sorted using a FACStar$^{Plus}$ based upon forward and side scatter gates to exclude dead cells which have lower forward and side scatter. The number of cells sorted was verified under the fluorescence microscope. Sorting efficiency was determined to be greater than 97%.

Example II

250 pre-B cells were sorted into 2.5 x 10$^8$ PBMC. The pre-B cells were stained with Hoechst 33 342 as in Example I and with monoclonal antibodies as described above. Sequential aliquots of cells were taken out of the staining solution just prior to analysis. The cells were washed twice, resuspended in PBS and were immediately analyzed on a FACStar$^{Plus}$ cells sorter which had been cleaned as described above. Cells were sorted onto a microscope slide if they were FITC$^+$ (i.e., FL1$^+$), PE$^+$ (i.e., FL2$^+$) and PerCp$^-$ (i.e., FL3$^-$). Cells were sorted onto a new slide after every 10$^6$ cells analyzed. Slides were examined under a UV fluorescence microscope. The presence of pre-B cells was verified on the slide by UV excitation. This experiment confirmed that the sort strategy worked.

Example III

0, 250, 2,500 and 25,000 pre-B cells were sorted into 2.5 x 10$^8$ PBMC. This represents a frequency of pre-B cells of 10$^{-6}$, 10$^{-5}$ and 10$^{-4}$ respectively. The pre-B cells were stained as in Example I with the first and second markers described above. The cells were washed twice, resuspended in PBS and were immediately analyzed on a FACStar$^{Plus}$ cells sorter which had been cleaned as described above.

Referring to FIG.s 3H and I respectively, gates were established in a plot of FL2 versus FL1 and FL3 versus FL1 for pre-B cells at a concentration of one in 10$^{-4}$ PBMC. The gates, referred to as "R5" in H and "R3" in I, were set so as to include the most likely areas were pre-B cells should occur.

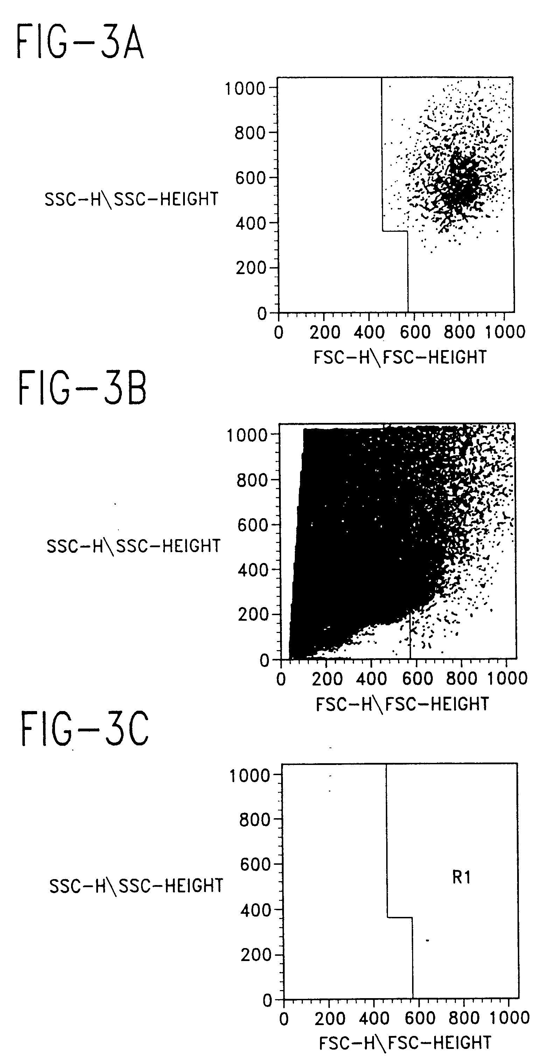

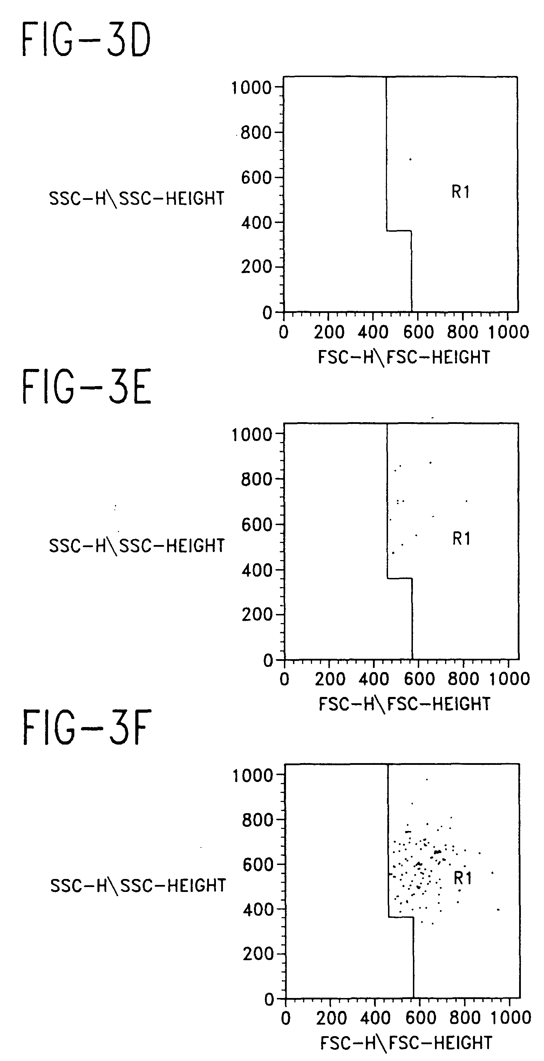

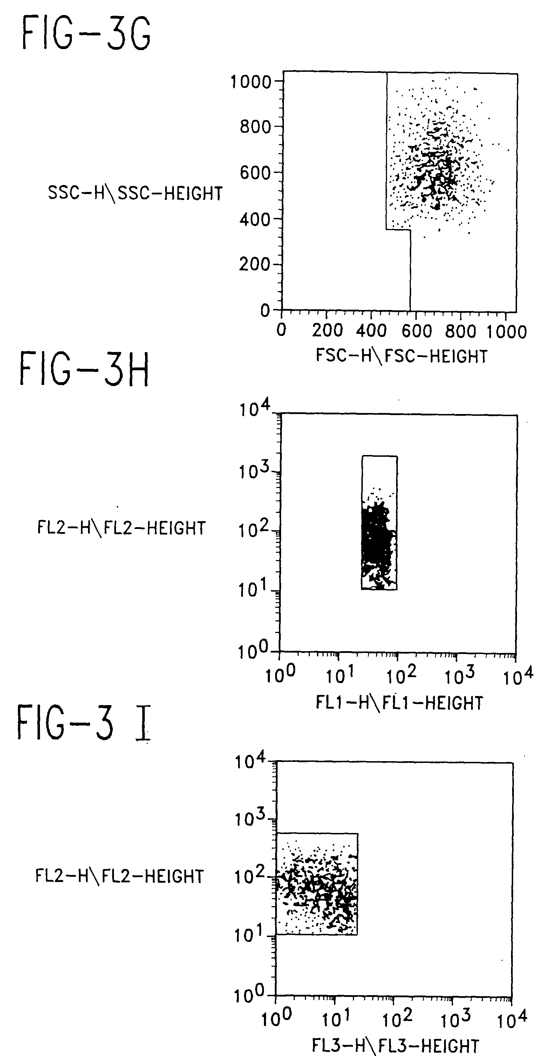

The R5 and R3 gates then were applied to the list mode data from the analysis, and a plot of SSC versus FSC was constructed. Referring to FIG. 3A, the gate "R1" was constructed in the scatter plot so as to include all of the pre-B cells. All three gates then were applied to various aliquots.

Referring to FIG. 3B and C respectively, PBMC were analyzed using anti-KLH as the marker without and with the R1, R3 and R5 gates. As can be seen, for ungated data numerous events are displayed in a plot of SSC versus FSC; however, when the gates are applied, only one event in 10$^6$ events analyzed is recorded.

Referring to FIG. 3D, when 0 pre-B cells are seeded into PBMC and 10$^6$ events are analyzed, only one event in 10$^6$ appears as background.

Referring to FIG.s 3E, F and G respectively, gates R1, R3 and R5 were applied to listmode data recorded from the analysis of 10$^{-6}$, 10$^{-5}$ and 10$^{-4}$ pre-B cells seeded into at least 10$^6$ PBMC. For 10$^{-6}$ seeded cells, 12-17 events were recovered in 4 replicate experiments. For 10$^{-5}$ seeded cells, 45-136 pre-B cells were recovered in 3 replicate experiments. For 10$^{-4}$ seeded cells, 371-1291 pre-B cells were recovered in 3 replicate experiments. As expected, the number of recovered cells increases by a factor of 10 as the number of seeded cells increases by a factor of 10.

**Claims**

1. A method for the analysis of particles in a sample at frequencies of less than one in 10$^4$ comprising the steps of:

   (a) combining the particles with at least one first marker, wherein said first marker is specific for the particle to be analyzed, and at least one second marker, wherein said second marker is specific for a majority of all other particles in the sample but not for the particle to be analyzed;
   (b) setting one or more fluorescence thresholds above the autofluorescence of the unlabelled particles in the sample so as to exclude from analysis all particles that do not exceed that threshold;
   (c) setting a gate for each of the light scatter, first marker and second marker parameters measured so as to include the rare events to be detected and exclude all other events; and
   (d) analyzing the particles in the sample for one or more light scatter parameters and for the presence of the first maker and absence of the second marker by means of flow cytometry within the gates set by step (c).

2. The method of claim 1 wherein the particles comprise cells in a biological fluid selected from the group consisting of blood, urine, lymph, bone marrow, ascites, cerebrospinal fluid and saliva.

3. The method of claim 1 wherein the particles comprise cells dis-aggregated from an organ or solid tissue.

4. The method of claim 1 wherein the particles comprise cells and the markers comprise immunofluorescence markers.

5. The method of claim 4 wherein the number of first markers is two and each marker is labelled with a fluorescent dye having an emission wavelength that is distinguishable from the other.

6. The method of claim 4 wherein the number of second markers is more than one and such marker is labelled with the same fluorescent dye, said dye having an emission wavelength that is distinguishable from the wavelength of the dye on the first marker.

7. The method of claim 4 wherein the number of first markers is two and the number of second markers is more than one wherein each of said markers is labelled with a fluorescent dye, said dyes on the first markers having an emission wavelength that is distinguishable from the wavelength of the dye on the second marker(s) and said dye on the second markers having an emission wavelength that is distinguishable from the wavelength of the dyes on the first markers.

8. The method of claim 1 wherein first marker is for a cancer cell and the second marker is CD34.

**Patentansprüche**

1. Verfahren für die Analyse von Teilchen in einer Probe mit Häufigkeiten von weniger als eins in $10^4$, umfassend die Stufen von:

   (a) Verbinden der Teilchen mit mindestens einem ersten Marker, wobei der erste Marker spezifisch für das zu analysierende Teilchen ist, und mindestens einem zweiten Marker, wobei der zweite Marker spezifisch für eine Mehrheit von allen anderen Teilchen in der Probe aber nicht für das zu analysierende Teilchen ist,

   (b) Festlegen eines oder mehrerer Fluoreszenzgrenzwerte über der Autofluoreszenz der unmarkierten Teilchen in der Probe, um von der Analyse alle Teilchen auszuschließen, die nicht jenen Grenzwert übersteigen;

   (c) Festsetzen einer Sperre für jeden der gemessenen Lichtstreuungs-, erster Marker- und zweiter Markerparameter, um so die seltenen nachzuweisenden Ereignisse einzuschließen und alle anderen Ereignisse auszuschließen, und

   (d) Analysieren der Teilchen in der Probe im Hinblick auf ein oder mehrere Lichtstreuungsparameter und im Hinblick auf das Vorhandensein des ersten Markers und Fehlens des zweiten Markers mittels Fließzytometrie in den von Stufe (c) gesetzten Sperren.

2. Verfahren nach Anspruch 1, wobei die Teilchen Zellen in einer biologischen Flüssigkeit, ausgewählt aus der Gruppe, bestehend aus Blut, Urin, Lymphe, Knochenmark, Ascites, Hirnflüssigkeit und Saliva, umfassen.

3. Verfahren nach Anspruch 1, wobei die Teilchen aufgeschlossene Zellen aus einem Organ oder festem Gewebe umfassen.

4. Verfahren nach Anspruch 1, wobei die Teilchen Zellen umfassen, und die Marker umfassen Immunfluoreszenzmarker.

5. Verfahren nach Anspruch 4, wobei die Zahl von ersten Markern zwei ist, und jeder Marker ist mit einem Fluoreszenzfarbstoff mit einer Emissionswellenlänge markiert, die von der anderen unterscheidbar ist

6. Verfahren nach Anspruch 4, wobei die Zahl von zweiten Markern mehr als eins ist, und ein derartiger Marker ist mit dem gleichen Fluoreszenzfarbstoff markiert, wobei der Farbstoff eine Emissionswellenlänge hat, die von der Wellenlänge des Farbstoffs auf dem ersten Marker unterscheidbar ist.

7. Verfahren nach Anspruch 4, wobei die Zahl von ersten Markern zwei ist, und die Zahl von zweiten Markern ist mehr als eins, wobei jeder der Marker mit einem Fluoreszenzfarbstoff markiert ist, wobei die Farbstoffe auf den ersten Markern eine Emissionswellenlänge haben, die unterscheidbar von der Wellenlänge des Farbstoffs auf dem (den) zweiten Marker(n) ist, und wobei der Farbstoff auf den zweiten Markern eine Emissionswellenlänge hat, die unterscheidbar von der Wellenlänge der Farbstoffe auf den ersten Markern ist.

8. Verfahren nach Anspruch 1, wobei erster Marker für eine Krebszelle ist, und der zweite Marker ist CD34.

**Revendications**

1. Procédé pour l'analyse de particules dans un échantillon à des fréquences inférieures à une pour $10^4$, comprenant les étapes consistant :

   (a) à combiner les particules avec au moins un premier marqueur, ledit premier marqueur étant spécifique pour la particule à analyser, et au moins un second marqueur, ledit second marqueur étant spécifique pour la majorité de toutes les autres particules dans l'échantillon mais pas pour la particule à analyser ;

   (b) à établir un ou plusieurs seuils de fluorescence au-dessus de l'autofluorescence des particules non marquées dans l'échantillon de façon à exclure de l'analyse toutes les particules qui ne dépassent pas ce seuil ;

(c) à établir une grille pour chacun des paramètres dispersion de lumière, premier marqueur et second marqueur mesurés de façon à inclure les événéments rares à détecter et à exclure tous les autres événements ; et

(d) à analyser les particules dans l'échantillon pour rechercher un ou plusieurs paramètres de dispersion de lumière et la présence du premier marqueur et l'absence du second marqueur au moyen d'une cytométrie de flux dans les grilles établies par l'étape (c).

2. Procédé selon la revendication 1, dans lequel les particules comprennent des cellules dans un fluide biologique choisi dans le groupe constitué par le sang, l'urine, la lymphe, la moelle osseuse, l'ascite, le liquide céphalorachidien et la salive,

3. Procédé selon la revendication 1, dans lequel les particules comprennent des cellules séparées d'un organe ou tissu solide.

4. Procédé selon la revendication 1, dans lequel les particules comprennent des cellules et les marqueurs comprennent des marqueurs d'immunofluorescence.

5. Procédé selon la revendication 4, dans lequel le nombre de premiers marqueurs est de deux et chaque marqueur est marqué avec un colorant fluorescent ayant une longueur d'onde d'émission qui est reconnaissable de l'autre.

6. Procédé selon la revendication 4, dans lequel le nombre de seconds marqueurs est supérieur à un et un tel marqueur est marqué avec le même colorant fluorescent, ledit colorant ayant une longueur d'onde d'émission qui est reconnaissable de la longueur d'onde du colorant sur le premier marqueur.

7. Procédé selon la revendication 4, dans lequel le nombre de premiers marqueurs est de deux et le nombre de seconds marqueurs est supérieur à un, dans lequel chacun desdits marqueurs est marqué avec un colorant fluorescent, lesdits colorants sur les premiers marqueurs ayant une longueur d'onde d'émission qui est reconnaissable de la longueur d'onde du colorant sur le(s) second(s) marqueur(s) et ledit colorant sur les seconds marqueurs ayant une longueur d'onde d'émission qui est reconnaissable de la longueur d'onde des colorants sur les premiers marqueurs,

8. Procédé selon la revendication 1, dans lequel le premier marqueur est pour une cellule cancéreuse et le second marqueur est CD34.

FIG-1

FIG-2A

FIG-2B

FIG-2C

## FIG-3A

## FIG-3B

## FIG-3C

FIG-3D

SSC-H\SSC-HEIGHT

R1

FSC-H\FSC-HEIGHT

FIG-3E

SSC-H\SSC-HEIGHT

R1

FSC-H\FSC-HEIGHT

FIG-3F

SSC-H\SSC-HEIGHT

R1

FSC-H\FSC-HEIGHT

FIG—3G

SSC—H\SSC—HEIGHT

FSC—H\FSC—HEIGHT

FIG—3H

FL2—H\FL2—HEIGHT

FL1—H\FL1—HEIGHT

FIG—3 I

FL2—H\FL2—HEIGHT

FL3—H\FL3—HEIGHT